Europäisches Patentamt

European Patent Office

Office européen des brevets

(11) Veröffentlichungsnummer: **0 297 407**
**A2**

## (12) EUROPÄISCHE PATENTANMELDUNG

(21) Anmeldenummer: **88109884.2**

(22) Anmeldetag: **22.06.88**

(51) Int. Cl.⁴: **G01K 5/22**

(30) Priorität: **26.06.87 DE 3721144**

(43) Veröffentlichungstag der Anmeldung:
**04.01.89 Patentblatt 89/01**

(84) Benannte Vertragsstaaten:
**AT BE CH ES FR GB IT LI LU NL SE**

(71) Anmelder: **Steinhilber, Helmut**
**Sonnenbergstrasse 40**
**CH-6052 Hergiswil(CH)**

(72) Erfinder: **Steinhilber, Helmut**
**Sonnenbergstrasse 40**
**CH-6052 Hergiswil(CH)**

(74) Vertreter: **Patentanwälte Dipl.-Ing. Klaus**
**Westphal Dr. rer. nat. Bernd Mussgnug Dr.**
**rer.nat. Otto Buchner**
**Waldstrasse 33**
**D-7730 VS-Villingen(DE)**

(54) **Vorrichtung zum Reinigen von Flüssigkeits-Fieberthermometern.**

(57) Zum Reinigen, Desinfizieren und Zurückschleudern von Quecksilber-Fieberthermometern (30) dient eine Vorrichtung, bei welcher die Thermometer (30) in einen rotierend angetriebenen Träger (18) eingesetzt werden. Die Thermometer (30) werden mittels des Trägers (18) durch ein flüssiges Reinigungs- und Desinfektionsmittel bewegt. Durch die Zentrifugalkraft werden bei der Rotation die Thermometer (30) gleichzeitig zurückgeschleudert.

Fig.1

## "Vorrichtung zum Reinigen von Flüssigkeits-Fieberthermometern"

Die Erfindung betrifft eine Vorrichtung zum Reinigen von Flüssigkeits-Fieberthermometern.

Flüssigkeits-Fieberthermometer, die in der Regel als Quecksilber-Fieberthermometer ausgebildet sind, weisen ein Ausdehnungsvolumen auf, das über eine Verengung mit einer Kapillare verbunden ist. Nach dem Gebrauch müssen die Thermometer gereinigt und vorzugsweise desinfiziert werden und ausserdem muß die Flüssigkeitssäule aus der Kapillare wieder in das Ausdehnungsvolumen zurückgeschleudert werden, um das Thermometer erneut einsatzbereit zu machen. In Krankenhäusern, wo bei einer gro Ben Zahl von Patienten gleichzeitig die Temperatur gemessen wird, ist das Reinigen und Zurückschleudern der Thermometer eine zeitraubende und lästige Tätigkeit.

Der Erfindung liegt die Aufgabe zugrunde, eine Vorrichtung zur Verfügung zu stellen, die ein automatisches Reinigen und Desinfizieren sowie Zurückschleudern der Thermometer ermöglicht. Diese Aufgabe wird erfindungsgemäß durch eine Vorrichtung mit den Merkmalen des kennzeichnenden Teils des Anspruchs 1 gelöst.

Vorteilhafte Ausführungsformen der Erfindung sind in den Unteransprüchen angegeben.

Die erfindungsgemäße Vorrichtung weist einen motorisch angetriebenen Träger auf, der eine größere Anzahl von Thermometern aufnehmen kann. Die Thermometer sind in dem rotierenden Träger so angeordnet, daß sich das Ausdehnungsvolumen auf einem größeren Radius befindet als die Kapillare, so daß die Quecksilbersäule bei der Rotation des Trägers durch die Zentrifugalkraft zurückgeschleudert wird. Weiter ist der Träger in einem Behälter angeordnet, der mit einem flüssigen Reinigungs-und Desinfektionsmittel so weit gefüllt werden kann, daß die Thermometer zumindest mit dem Teil, welcher mit dem Patienten in Berührung kommt, in dieses Reinigungs-und Desinfektionsmittel eintauchen. Bei der Rotation des Trägers werden die Thermometer durch das Reinigungs- und Desinfektionsmittel bewegt und dadurch gespült und desinfiziert.

Vorzugsweise sind der Träger und der Flüssigkeitsspiegel des Reinigungs- und Desinfektionsmittels in der Höhe gegeneinander verstellbar. Dadurch ist eine Stellung möglich, in welcher die in dem Träger angeordneten Thermometer in das Reinigungs- und Desinfektionsmittel eintauchen, um die Thermometer zu spülen. In einer anderen Stellung tauchen die Thermometer nicht mehr in das Reinigungs- und Desinfektionsmittel ein, damit dieses bei der Rotation des Trägers von den Thermometern abgeschüttelt wird, so daß die Thermometer schnell trocknen. Vorzugsweise ist für den Antrieb des Trägers ein Elektromotor vorgesehen, der zwischen zwei Drehzahlen umschaltbar ist. Beim Eintauchen der Thermometer in das Reinigungsmittel ist eine niedrigere Drehzahl vorgesehen, während die höhere Drehzahl dazu verwendet wird, die Thermometer ausserhalb des Reinigungsmittels zu trocknen und zurückzuschleudern.

Der Träger ist vorzugsweise ein einfaches kegelförmig ausgebildetes Kunststoffteil, das auf seinem Umfang in der Kegelmantellinie verlaufende Aufnahmen für die Thermometer aufweist. Diese Aufnahmen sind in einfachster Weise als Rinnen ausgebildet, wobei am oberen und unteren Ende jeder dieser Rinnen eine Öffnung vorgesehen ist, die das eingesetzte Thermometer umschließt und dieses bei der Rotation des Trägers gegen die Zentrifugalkraft festhält.

Die gegenseitige Höhenverstellung von Träger und Flüssigkeitsspiegel des Reinigungs- und Desinfektionsmittels kann in unterschiedlicher Weise realisiert werden.

Der Träger kann in dem Behälter in der Höhe unveränderbar angeordnet sein, während der Flüssigkeitsspiegel des Reinigungssmittels angehoben und abgesenkt wird. Ebenso ist es möglich, den Flüssigkeitsspiegel des Reinigungsmittels in dem Behälter festzuhalten und die axiale Höhenlage des Trägers in dem Behälter zu ändern.

In einer Ausführungsform ist der Träger axial in dem Behälter festgelegt und der Behälter sitzt höhenverstellbar auf einem Gehäuseunterteil, welches das flüssige Reinigungs- und Desinfektionsmittel aufnimmt. Wird der Behälter gegen das Gehäuseunterteil abgesenkt, so dringt das Reinigungsmittel in den Behälter ein, so daß die vom Träger aufgenommenen Thermometer in dieses Reinigungsmittel eintauchen. Wird der Behälter dagegen in Bezug auf das Gehäuseunterteil angehoben, so gelangt das Reinigungsmittel wieder vollständig in das Gehäuseunterteil.

Das flüssige Reinigungs- und Desinfektionsmittel kann dabei in einem flexiblen Beutel aufgenommen sein, der sich in dem Gehäuseunterteil befindet und an eine Bodenöffnung des Behälters angeschlossen wird. Wird der Behälter gegen das Gehäuseunterteil abgesenkt, so drückt er den flexiblen Beutel zusammen und preßt das Reinigungsmittel aus dem Beutel in den Behälter. Beim Anheben des Behälters fließt das Reinigungsmittel wieder in den Beutel zurück. Muß das Reinigungs- und Desinfektionsmittel erneuert werden, so muß nur der Beutel ausgetauscht werden. Das Reinigungs- und Desinfektionsmittel kann in dem Beutel verpackt geliefert und entsorgt werden.

Das Gehäuseunterteil kann ebenso auch als Wanne ausgebildet sein, die das flüssige Reinigungs- und Desinfektionsmittel aufnimmt. Der Behälter, in welchem der rotierenden Träger axial fest angeordnet ist, weist einen durchbrochenen Boden auf und wird in das Gehäuseunterteil abgesenkt, so daß das Reinigungsmittel in den Behälter bis zu der erforderlichen Höhe eindringt. Wird der Behälter in dem Gehäuseunterteil angehoben, so sinkt der Flüssigkeitsspiegel des Reinigungsmittels in dem Behälter ab.

Es ist auch möglich, das flüssige Reinigungs- und Desinfektionsmittel unmittelbar in den Behälter mit geschlossenem Boden einzufüllen, so daß der Flüssigkeitsspiegel des Reinigungsmittels in dem Behälter stets auf gleicher Höhe bleibt. In dieser Ausführung kann der die Thermometer aufnehmende rotierende Träger in zwei unterschiedlichen Höhenstellungen auf der Antriebswelle festgelegt werden, wobei in einer Höhenstellung die Thermometer in das Reinigungsmittel eintauchen, während sie sich in der anderen Höhenstellung oberhalb des Flüssigkeitsspiegels des Reinigungsmittels befinden.

Um die Höhenverstellung in den verschiedenen Ausführungsformen zu bewirken, sind vorzugsweise Vorsprünge über den Umfang verteilt, die einen gegenseitigen Winkelabstand aufweisen. Wenn der Behälter gegenüber einem Gehäuseunterteil verstellbar ist, sind sowohl an dem Behälter als auch an dem Gehäuseunterteil solche Vorsprünge vorgesehen. Durch Winkelverdrehung kann der Behälter so gegenüber dem Gehäuseunterteil angeordnet werden, daß sich die Vorsprünge des Behälters auf den Vorsprüngen des Gehäuseunterteils abstützen und der Behälter dadurch in der angehobenen Stellung gehalten wird. In einer anderen Drehstellung des Behälters gelangen die Vorsprünge des Behälters in den Winkelzwischenraum zwischen den Vorsprüngen des Gehäuseunterteils, so daß der Behälter abgesenkt werden kann.

Ist der Träger höhenverstellbar in dem Behälter angeordnet, so ist er vorzugsweise in zwei unterschiedlichen Höhenstellungen auf der Antriebswelle festlegbar. Hierzu kann beispielsweise die Antriebswelle als Mehrkantwelle ausgebildet sein, auf die der Träger mit einer entsprechenden Mehrkantbuchse aufsteckbar ist. Die Mehrkantflächen der Antriebswelle weisen alternierend in unterschiedlicher axialer Höhe angeordnete Eingriffsaufnahmen auf, in welche der Träger eingreifen kann. Durch Verdrehen des Trägers gegenüber der Antriebswelle um eine Mehrkantteilung kommt der Träger mit den oberen oder den unteren Eingriffsaufnahmen in Eingriff und befindet sich in der angehobenen oder der abgesenkten Stellung, wobei durch den Mehrkant die drehschlüssige Antriebsverbindung in beiden Fällen bewirkt wird. Die Eingriffsaufnahmen können dabei auch als zwei unter 90° zu einander angeordnete axiale Schlitze unterschiedlicher axialer Tiefe in der Antriebswelle ausgebildet sein, in welcher ein die Aufsteckbuchse des Trägers diametral durchsetzender Stift eingreift. Da dieser Stift dabei auch als Mitnehmer wirkt, kann die Antriebswelle kreisförmigen Querschnitt aufweisen.

Zum Ein- und Ausschalten des antreibenden Elektromotors ist ein Schalter vorgesehen, der in dem Gehäuseunterteil oder bei höhenverstellbarem Träger in dem Behälter angeordnet ist. Der Schalter dient vorzugsweise auch zum Umschalten zwischen zwei Drehzahlen. In der Ausführungsform mit gegen das Gehäuseunterteil absenkbarem Behälter kann der Schalter auch als Mikroschalter ausgebildet sein, der durch den Behälter betätigt wird, wenn dieser in die abgesenkte Stellung bewegt wird. Die Vorrichtung wird dadurch automatisch in Betrieb gesetzt, wenn der Behälter abgesenkt wird und die Thermometer in das Reinigungs- und Desinfektionsmittel eintauchen. Wird der Behälter nach der Reinigung wieder angehoben, so wird der Mikroschalter wieder freigegeben. Dabei ist eine Nachlaufschaltung vorgesehen, die den Elektromotor erst nach einer gewissen Nachlaufzeit zum Trocknen der Thermometer abschaltet. Vorzugsweise wird während dieser Nachlaufzeit die Drehzahl des Elektromotors erhöht.

Im folgenden wird die Erfindung anhand eines in der Zeichnung dargestellten Ausführungsbeispiels näher erläutert. Es zeigen,

Figur 1 - einen vertikalen Axialschnitt durch die Vorrichtung,

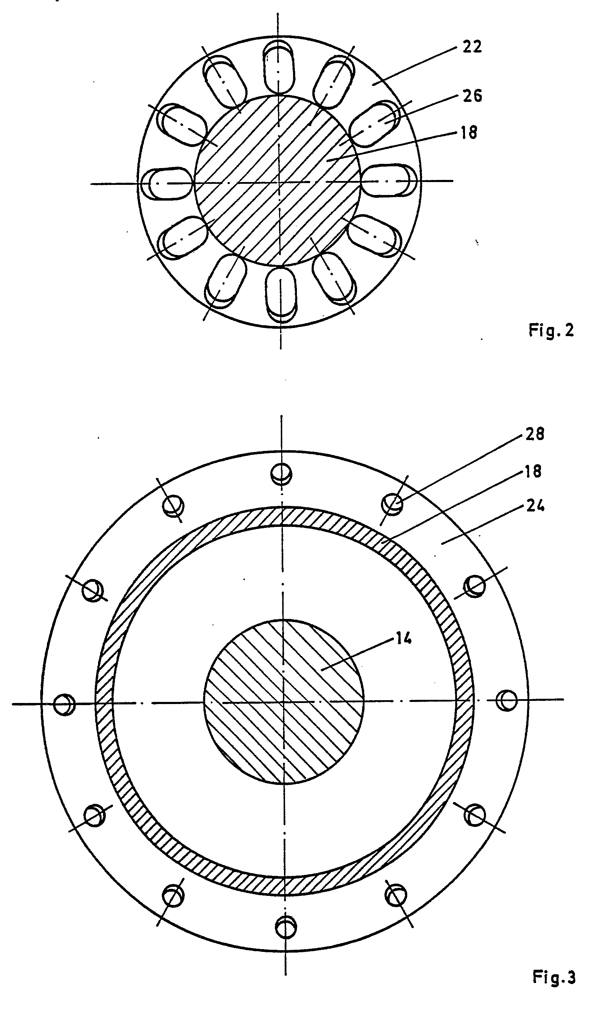

Figur 2 - einen horizontalen Querschnitt gemäß der Linie II-II in Figur 1,

Figur 3 - einen Querschnitt gemäß der Linie III-III in Figur 1,

Figur 4 - einen Querschnitt gemäß der Linie IV-IV in Figur 1,

Figur 5 - einen Ausschitt aus Figur 1 in der angehobenen Stellung des Behälters und

Figur 6 - einen entsprechenden Ausschnitt in der abgesenkten Stellung des Behälters.

Die Vorrichtung weist einen topfförmigen Behälter 10 aus Kunststoff auf, der durch einen Deckel 12 verschließbar ist. Zentrisch auf dem Boden des Behälters 10 ist ein Elektromotor 14 angeordnet, dessen Welle als Antriebswelle 16 vertikal nach oben ragt.

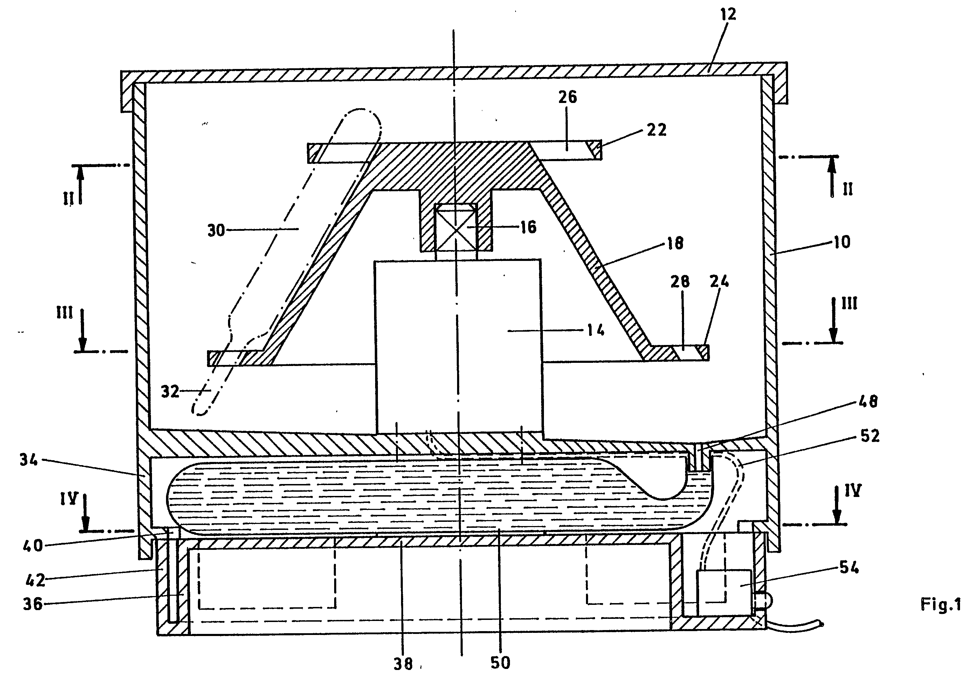

Auf der mit einem Vierkant versehenen Antriebswelle 16 sitzt ein aus Kunststoff gespritzter Träger 18. Der Träger 18 hat die Form eines sich nach unten erweiternden, den Elektromotor 14 übergreifenden Kegelstumpfes. Über den Mantelumfang des Trägers 18 sind in gleichem Winkelabstand in der mantellinie verlaufende Aufnahmen für Quecksilber-Fieberthermometer vorgesehen. Am oberen und unteren Ende des Trägers 18 ist ein scheibenförmiger nach aussen ragender Flansch 22 bzw. 24 angeformt. Der obere Flansch 22 und der untere Flansch 24 weisen jeweils Öffnungen 26 bzw. 28 auf, die in der Mantellinie fluchten. Die Öffnungen 26 des oberen Flansches 22 haben eine ovale Form, während die Öffnungen 28 des unteren Flansches 24 rund sind. Die oberen Öffnungen 26 entsprechend im wesentlichen der Aussenkontur eines Quecksilber-Fieberthermometers 30, wärend die unteren Öffnungen 28 im wesentlichen dem Querschnitt der das Quecksilber-Ausdehnungsvolumen 32 des Thermometers aufnehmenden Spitze entsprechen. In den Träger 18 können auf diese Weise mehrere Thermometer 30 (im dargestellten Ausführungsbeispiel 12 Thermometer) eingesetzt werden, wie dies in Figur 1 angedeutet ist. Die das Ausdehnungsvolumen 32 aufnehmende Spitze ist dabei in die Öffnung 28 des unteren Flansches 24 eingesetzt, während die Öffnung 26 des oberen Flansches 22 den Kapillaren-Teil des Thermometers 30 umschließt. Das Thermometer 30 kann durch die obere Öffnung 26 von oben in den Träger 18 eingesetzt und aus diesem entnommen werden. Da wegen der kegelförmigen Ausbildung des Trägers 18 die oberen Enden des Thermometer 30 verhältnismäßig dicht beieinander sind, kann der Träger 18 gegebenenfalls auch so ausgebildet sein, daß die Thermometer 30 jeweils abwechselnd axial höher und tiefer in dem Träger 18 sitzen. Dies erleichtert das Erfassen der oberen Enden der Thermometer 30.

Der Behälter 10 weist einen nach unten überstehenden Rand 24 auf, mit welchem er ein Gehäuseunterteil 36 übergreift, dessen Höhe im wesentlichen der Höhe des Randes 34 entspricht. Das Gehäuseunterteil 36 ist kreisförmig ausgebildet und weist einen obere kreisscheibenförmige Auflageplatte 38 auf.

Etwas oberhalb der Unterkante des Randes 34 ragen von diesem Rand 34 Vorsprünge 40 nach Innen, die über den Umfang verteils sind und einen gegenseitigen Winkelabstand aufweisen. Im Ausführungsbeispiel sind vier unter 90° gegeneinander versetzte Vorsprünge 40 vorgesehen. Der nach oben gerichtete Rand 42 des Gehäuseunterteils 36 weist entsprechende nach oben gerichtete Vorsprünge 44 auf, die sich auf demselben Radius wie die Vorsprünge 40 befinden. Die Vorsprünge 44 sind in gleicher Anzahl und Winkelanordnung über den Umfang verteilt wie die Vorsprünge 40. Zwischen den Vorsprüngen 44 des Gehäuseunterteils 36 bleiben Aussparungen 46 des Randes 42 frei, deren Winkelbreite größer ist als die Winkelbreite der Vorsprünge 40 des Randes 34 des Behälters 10.

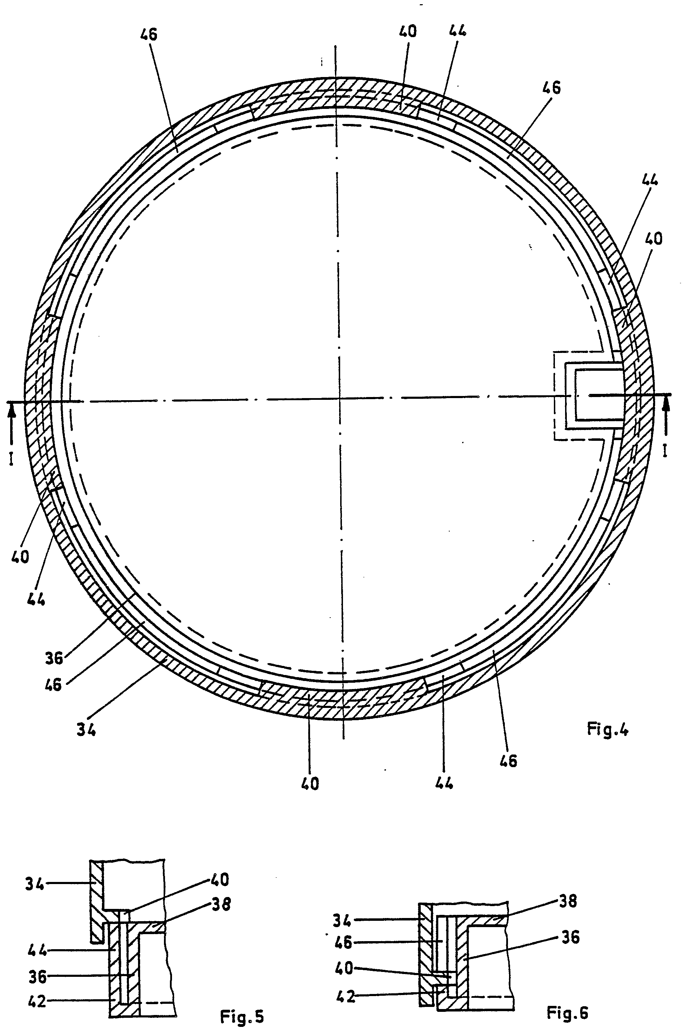

Wird der Behälter 10 in seiner Winkelstellung gegenüber dem Gehäuseunterteil 36 so angeordnet, daß die Vorsprünge 40 die Vorsprünge 44 überlappen, so stützt sich der Behälter 10 mit seinen Vorsprüngen 40 auf den Vorsprüngen 44 des Gehäuseunterteils 36 ab und der Behälter 10 nimmt eine angehobene Stellung ein. Diese Stellung ist in den Figuren 1, 4 und 5 dargestellt. In dieser angehobenen Stellung ergibt sich ein freier Raum zwischen dem Boden des Behälters 10 und der Auflageplatte 38 des Gehäuseunterteils 36, wie dies in Figur 1 zu erkennen ist. Wird der Behälter 10 gegenüber dem Gehäuseunterteil 36 aus der in Figur 4 dargestellten Stellung um 45° gedreht, so gelangen die Vorsprünge 40 des Behälters 10 in den Bereich der Aussparungen 46 des Randes 42 des Gehäuseunterteils 36 und der Behälter 10 kann nach unten abgesenkt werden, wobei sich der Boden des Behälters auf die Auflageplatte 38 des Gehäuseunterteils 36 absenkt. Das Eingreifen der Vorsprünge 40 in Aussparungen 46 ist in Figur 6 dargestellt.

Wie Figur 1 zeigt, weist der Boden des Behälters 10 ein leichtes Gefälle auf, das zu einer Eintrittsöffnung 48 verläuft, die an der Unterseite des Bodens mit einem Stutzen versehen ist. Auf diesen Stutzen ist ein Beutel 50 aus einer flexiblen Kunststoffolie aufsteckbar, der zwischen dem Boden des Behälters 10 und der Auflageplatte 38 des Gehäuseunterteils 36 angeordnet ist. Der Beutel 50 ist mit einem flüssigen Reinigungs- und Desinfektionsmittel gefüllt.

Befindet sich der Behälter 10 in seiner angehobenen Stellung, wie dies in Figur 1 dargestellt ist, so befindet sich das Reinigungsmittel in dem Beutel 50. Wird der Behälter 10 abgesenkt, so drückt er durch sein Eigengewicht das flüssige Reinigungs- und Desinfektionsmittel aus dem Beutel 50 über die Eintrittsöffnung 48 in den Behälter 10. Ist der Behälter 10 vollständig abgesenkt, so ist der Flüssigkeitsspiegel des Reinigungsmittels in dem Behälter 10 soweit angestiegen, daß die in den Träger 18 eingesetzten Thermometer 30 zumindest mit ihrer unteren Hälfte in das Reinigungsmittel eintauchen. Wird der Behälter 10 wieder angehoben, so fließt das Reinigungsmittel wieder in den Beutel 50 zurück.

Der Elektromotor 14 ist über ein Kabel 52 und einen Ein-Aus-Schalter 54 an das Stromnetz anschließbar. Der Schalter 54 ist als Mikroschalter ausgebildet, der durch den Rand 34 des Behälters 10 betätigt wird und den Elektromotor einschaltet, wenn der Behälter 10 abgesenkt wird. Der Schalter 54 ist ausserdem mit einer Nachlaufschaltung ausgestattet, die ein Nachlaufen des Elektromotors 14 gewährleistet, wenn der Behälter 10 wieder angehoben wird. Vorzugsweise kann diese Nachlaufschaltung mit einer Erhöhung der Drehzahl des Elektromotors verbunden sein.

Bei angehobenem Behälter 10 werden die gebrauchten Thermometer 30 in den Träger 18 eingesetzt. Dann wird der Behälter 10 durch Verdrehen gegenüber dem Gehäuseunterteil 36 abgesenkt, wobei des flüssige Reinigungs-und Desinfektionsmittel aus dem Beutel 50 in den Behälter 10 eintritt. Der abgesenkte Behälter 10 betätigt den Schalter 54, so daß der Träger 18 mit niedriger Drehzahl rotiert und die Thermometer 30 durch das Reinigungs- und Desinfektionsmittel bewegt, um diese abzuspülen und zu desinfizieren. Anschließend wird der Behälter 10 angehoben und wieder so verdreht, daß er in der angehobenen Stellung aufsitzt. Das Reinigungsmittel fließt wieder in den Beutel 50 zurück und der Träger 18 rotiert aufgrund der Nachlaufschaltung noch für eine gewisse Zeit mit erhöhter Drehzahl. Dabei wird das flüssige Reinigungs- und Desinfektionsmittel von den Thermometern 30 abgeschüttelt, um diese zu trocknen. Gleichzeitig wird die Quecksilbersäule der Thermometer 30 durch die Zentrifugalkraft in das Ausdehnungsvolumen 32 zurückgeschüttelt. Die Thermometer 30 können dann wieder gebrauchsfertig entnommen werden. Zum Austau - schen des Reinigungs- und Desinfektionsmittels muß nur der Beutel 50 von dem Stutzen der Eintrittsöffnung 48 abgezogen und durch einen neuen Beutel 50 ersetzt werden.

**Ansprüche**

1. Vorrichtung zum Reinigen von Flüssigkeits-Fieberthermometern, dadurch gekennzeichnet, daß in einem mit einem flüssigen Reinigungs- und Desinfektionsmittel füllbaren Behälter (10) ein rotierend antreibbarer, die Thermometer (30) aufnehmender Träger (18) angeordnet ist und daß das Ausdehnungsvolumen (32) der in dem Träger (18) aufgenommenen Thermometer (30) sich auf einem größeren Radius bezüglich der Rotationsachse des Trägers (18) als deren Kapillare und in dem mit dem Reinigungs- und Desinfektionsmittel füllbaren Bereich des Behälters (10) befindet.

2. Vorrichtung nach Anspruch 1, dadurch gekennzeichnet, daß der Flüssigkeitsspiegel des Reinigungs- und Desinfektionsmittels und der Träger (18) in der Höhe gegeneinander verstellbar sind, wobei in einer Stellung die in dem Träger (18) aufgenommenen Thermometer (30) mindestens teilweise in das Reinigungs- und Desinfektionsmittel eintauchen und in einer anderen Stellung sich ausserhalb des Reinigungs- und Desinfektionsmittels befinden.

3. Vorichtung nach Anspruch 1 oder 2, dadurch gekennzeichnet, daß die Lage der Thermometer (30) in dem Träger (18) den Mantellinien eines sich nach unten erweiternden Kegels entspricht, wobei das Ausdehnungsvolumen (32) der Thermometer (30) unten angeordnet ist.

4. Vorrichtung nach den Ansprüchen 2 und 3, dadurch gekennzeichnet, daß der Träger (18) kegelförmig ausgebildet ist, auf einer koaxialen Antriebswelle (16) sitzt und über seinen Umfang verteilte, in der Kegelmantellinie verlaufende Aufnahmen für die Thermometer (30) aufweist.

5. Vorrichtung nach Anspruch 4, dadurch gekennzeichnet, daß die Aufnahmen jeweils an ihrem oberem und unterem Ende eine das aufgenommene Thermometer (30) umschließende Öffnung (26 bzw. 28) aufweisen.

6. Vorrichtung nach Anspruch 2, dadurch gekennzeichnet, daß der Träger (18) axial fest in dem Behälter (10) angeordnet ist, daß das Reinigungs- und Desinfektionsmittel in einem Gehäuseunterteil (36) aufgenommen ist und daß der Behälter (10) gegen das Gehäuseunterteil (36) absenkbar ist, wobei das Reinigungs- und Desinfektionsmittel in den Behälter (10) eintritt.

7. Vorrichtung nach Anspruch 6, dadurch gekennzeichnet,

7. Vorrichtung nach Anspruch 6, dadurch gekennzeichnet, daß das Reinigungs- und Desinfektionsmittel in einem flexiblen Beutel (50) in dem Gehäuseunterteil (36) aufgenommen ist, daß der Beutel (50) an eine Eintrittsöffnung (48) im Boden des Behälters (10) anschließbar ist und daß der Beutel (50) durch den abgesenkten Behälter (10) zusammendrückbar ist.

8. Vorrichtung nach Anspruch 6, dadurch gekennzeichnet, daß das Gehäuseunterteil (36) als das Reinigungs-und Desinfektionsmittel aufnehmende Wanne ausgebildet ist, in welche der mit durchbrochenem Boden ausgebildete Behälter (10) absenkbar ist.

9. Vorrichtung nach einem der Ansprüche 6 bis 8, dadurch gekennzeichnet, daß der Behälter (10) und das Gehäuseunterteil (36) jeweils über den Umfang verteilte, im Winkel beabstandete Vorsprünge (40 bzw. 44) aufweisen, daß in einer gegenseitigen Winkelstellung von Behälter (10) und Gehäuseunterteil (36) sich deren Vorsprünge im Winkel überlappen, wodurch der Behälter (10) sich in seiner angehobenen Stellung über die Vorsprünge (40, 44) auf dem Gehäuseunterteil (36) stützt und daß in einer dagegen verdrehten Winkelstellung von Behälter (10) und Gehäuseunterteil (36) sich deren Vorsprünge (40 bzw. 44 ) nicht überlappen, wodurch sich der Behälter (10) gegen das Gehäuseunterteil (36) absenkt.

10.Vorrichtung nach Anspruch 2, dadurch gekennzeichnet, daß der Träger (18) auf einer in dem Behälter (10) angeordneten Antriebswelle (16) in einer angehobenen und einer abgesenkten Stellung drehschlüssig befestigbar ist.

11.Vorrichtung nach Anspruch 10, dadurch gekennzeichnet, daß die Antriebswelle (16) im Winkel gegeneinander versetzte und in unterschiedlicher Höhe angeordnete axiale Eingriffsaufnahmen aufweist, in welche der Träger (18) entsprechend seiner Winkelstellung gegenüber der Antriebswelle wahlweise drehschlüssig eingreift.

12.Vorrichtung nach einem der vorhergehenden Ansprüche, dadurch gekennzeichnet, daß der Träger (18) mittels eines Elektromotors (14) umschaltbar mit zwei Rotationsgeschwindigkeiten antreibbar ist.

Fig.1

22

26

18

Fig. 2

28

18

24

14

Fig.3

Fig.4

Fig.5

Fig.6